# FASCICULE DE BREVET EUROPEEN

(11) **EP 0 751 150 B1**
(45) Date de publication et mention de la délivrance du brevet: **05.06.2002**
(21) Numéro de dépôt: 96401426.0
(22) Date de dépôt: 27.06.1996
(51) Int. Cl.: C08B 37/16, A61K 47/48, A61K 9/127

(54) **Dérivés de cyclodextrines, leur préparation et leur utilisation pour incorporer des molecules hydrophobes dans des systèmes de tensioactifs organisés**
Cyclodextrinderivate, ihre Herstellung und Verwendung zur Einbringung von hydrophoben Molekülen in strukturierten Tensidsystemen
Cyclodextrin derivatives, their preparation and their use to incorporate hydrophobic molecules in organized surfactant systems

(30) Priorité: 29.06.1995 FR 9507841
(43) Date de publication de la demande: 02.01.1997
(73) Titulaire: COMMISSARIAT A L'ENERGIE ATOMIQUE, 75015 Paris (FR)
(72) Inventeur: Djedaini-Pilard, Florence, 91150 Etampes (FR); Lin, Jing, 91600 Savigny-sur-Orge (FR); Perly, Bruno, 78320 La Verriere (FR)
(74) Mandataire: Dubois-Chabert, Guy

(56) Documents cités:
- WO-A-90/02141
- WO-A-95/15746

## Description

La présente invention a pour objet de nouveaux dérivés de cyclodextrines, utilisables en particulier pour l'incorporation en milieu aqueux de composés chimiques hydrophobes tels que des molécules pharmaceutiquement actives.

De façon plus précise, elle concerne des dérivés amphiphiles de cyclodextrines susceptibles de permettre, d'une part, l'inclusion de molécules hydrophobes dans la cyclodextrine pour former un complexe d'inclusion et, d'autre part, l'incorporation de tels complexes dans des systèmes de tensioactifs organisés tels que des petites vésicules phospholipidiques.

Cette incorporation est destinée à permettre le transport de la molécule hydrophobe, par exemple d'un principe actif, en particulier par voie transmembranaire, par exemple transdermique.

Les cyclodextrines ou cyclomaltooligosac-charides sont des composés d'origine naturelle formés par l'enchaînement de 6, 7 ou 8 unités glucose liées en α 1→4. De nombreux travaux ont montré que ces cyclodextrines pouvaient former des complexes d'inclusion avec des molécules hydrophobes et permettre ainsi la solubilisation de ces molécules dans des milieux aqueux. De nombreuses applications ont été proposées pour tirer profit de ce phénomène, en particulier dans le domaine pharmaceutique, comme il est décrit par D. Duchêne dans l'ouvrage intitulé "Cyclodextrins and their industrial uses", chapitre 6, page 213 à 257, Editions de Santé, 1987. Des compositions pharmaceutiques utilisant des cyclodextrines ont d'ailleurs été commercialisées au Japon, en Italie et plus récemment en France, par exemple par Pierre Fabre Médicament pour le Brexin® qui est un complexe d'inclusion du Piroxicam dans la β-cyclodextrine.

Parmi les cyclodextrines utilisables, la β-cyclodextrine qui comporte 7 unités glucose, est la plus adaptée au niveau de la taille de sa cavité et elle est la moins coûteuse des trois. De nombreuses modifications chimiques de la β-cyclodextrine ont été décrites afin de la rendre amphiphile dans le but de l'incorporer à des systèmes organisés.

Ainsi, L. Jullien et coll ont décrit dans J. Chem. Soc. Perkin Trans 2, 1993, p. 1011-1020, des dérivés de β-cyclodextrine comportant des chaînes aliphatiques situées en positions primaire et secondaire, en vue d'incorporer ces dérivés de cyclodextrines dans des vésicules de phosphatidylcholine. Ces dérivés sont amphiphiles et peuvent donc être incorporés dans les vésicules, mais la cavité interne de la cyclodextrine n'est plus accessible en raison du fort encombrement stérique des chaînes aliphatiques. Par conséquent, ces dérivés sont incapables d'inclure des molécules hydrophobes, en particulier des molécules de principe actif.

N. Bellanger et B. Perly ont décrit dans J. Mol. Struc., 1992, 273, pages 215-226, des dérivés aminés de cyclodextrines comportant une seule chaîne aliphatique sur le groupe amine de la mono-6-désoxy-6-amino-cyclodextrine, obtenus par réaction du dérivé aminé avec un acide carboxylique. Bien que ces dérivés ne comportent qu'une seule chaîne aliphatique, ils présentent l'inconvénient de conduire à une auto-inclusion de la chaîne aliphatique dans la cyclodextrine, ce qui ne permet ni l'incorporation à de petites vésicules de phospholipides, ni l'inclusion de molécules hydrophobes dans la cavité interne de la cyclodextrine.

Le document WO/02141 décrit de nombreux dérivés de cyclodextrine substitués par un groupe amino, par exemple un groupe aminoalkylamino, en vue de les lier de façon covalente à des agents tels que des produits pharmaceutiques.

Le document WO95/15746 décrit des liposomes unilamellaires dans lesquels peuvent être introduits des complexes d'inclusion de dérivés de cyclodextrine tels que des dérivés amino.

La présente invention a précisément pour objet d'autres dérivés de cyclodextrines, qui comportent une ou plusieurs chaînes aliphatiques leur conférant des propriétés amphiphiles, sans conduire pour autant au phénomène d'auto-inclusion de la ou des chaînes dans la cyclodextrine. De ce fait, on peut obtenir à partir de tels dérivés des complexes d'inclusion contenant une molécule hydrophobe et l'incorporation de ces complexes dans des vésicules de phospholipides.

Selon l'invention, le dérivé de cyclodextrine répond à la formule : 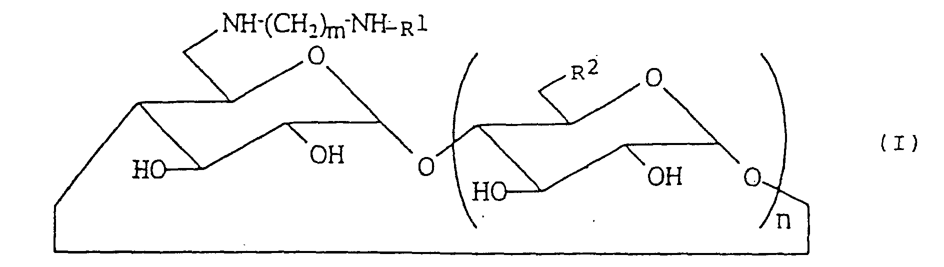 dans laquelle R¹ représente un groupe protecteur d'une fonction amine, les R² qui peuvent être identiques ou différents, représentent OH ou NH-(CH₂)ₘ-NHR¹, m est un nombre entier allant de 2 à 18, et n est égal à 5, 6 ou 7.

Dans ce dérivé, on obtient les propriétés amphiphiles grâce au greffage d'au moins une chaîne aliphatique (CH₂)ₘ-NH-R¹ qui ne donne pas lieu à un phénomène d'auto-inclusion lorsque le dérivé de cyclodextrine est en milieu aqueux. De ce fait, on peut obtenir à partir de ce dérivé un complexe d'inclusion par incorporation d'une molécule hydrophobe dans la cavité interne de la cyclodextrine, et incorporer ce complexe d'inclusion dans des petites vésicules de phospholipides.

De préférence, le dérivé de cyclodextrine comporte une seule chaîne aliphatique, tous les R² représentant OH.

Dans le dérivé de cyclodextrine de l'invention, le groupe R¹ joue le rôle de groupe protecteur de la fonction amine NH₂. Pour remplir ce rôle, R¹ peut représenter le groupe nitrophénylsulfényle, le groupe fluorène-9-yl méthoxycarbonyle ou un groupe de formule COOR³ dans laquelle R³ peut être un groupe alkyle, linéaire ou ramifié, ou aryle éventuellement substitués.

Lorsqu'on utilise un groupe alkyle, celui-ci a généralement de 4 à 18 atomes de carbone, et il est de préférence ramifié.

Le groupe aryle peut être par exemple le groupe benzyle.

Avantageusement R³ représente le groupe tert-butyle.

Les dérivés de cyclodextrine de l'invention peuvent être des dérivés de l'α-, β- ou γ-cyclodextrine. De préférence, on utilise les dérivés de la β-cyclodextrine, ce qui correspond dans la formule (I) donnée ci-dessus au cas où n est égal à 6.

La chaîne aliphatique du dérivé de cyclodextrine de l'invention peut comporter entre les deux groupes amino, 2 à 18 atomes de carbone. On obtient en particulier de bons résultats, lorsque m est compris entre 6 et 12, par exemple égal à 6.

Les dérivés de cyclodextrine de l'invention peuvent être préparés par des procédés classiques. Ainsi, on peut préparer les dérivés de formule (I) dans laquelle tous les R² représentent OH, à partir des dérivés tosylés ou naphtosulfonylés correspondants.

Aussi, l'invention a également pour objet un procédé de préparation du dérivé de cyclodextrine répondant à la formule (I) donnée ci-dessus dans laquelle tous les R² représentent OH, qui comprend les étapes suivantes :
a) faire réagir un dérivé de cyclodextrine de formule : 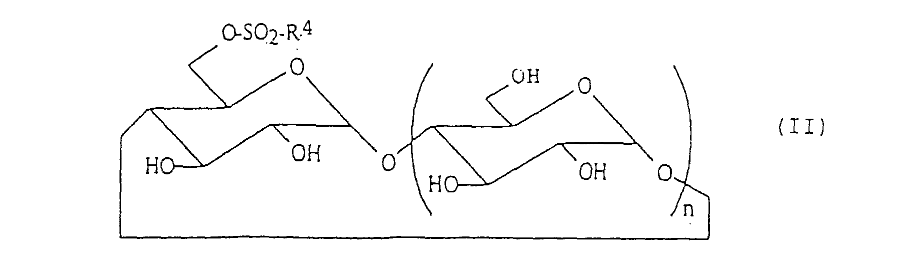 dans laquelle R⁴ est un groupe p-tolyle ou naphtyle, et n est égal à 5, 6 ou 7, avec un diaminoalcane de formule NH₂-(CH₂)ₘ-NH₂ dans laquelle m a la signification donnée ci-dessus, pour obtenir un dérivé de cyclodextrine de formule : 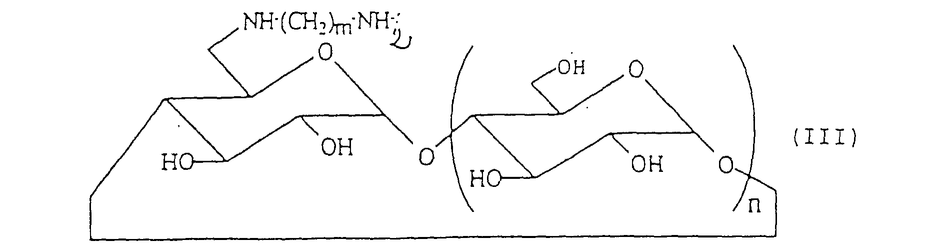 dans laquelle n et m sont tels que définis ci-dessus, et
b) faire réagir le dérivé de formule (III) avec un réactif comportant le groupe protecteur R¹ pour obtenir le dérivé de cyclodextrine de formule (I).

Dans l'étape a) de ce procédé, on fait réagir le dérivé de cyclodextrine de formule (II) avec le diaminoalcane voulu dans un solvant organique tel que le diméthyl formamide (DMF) en présence d'une amine tertiaire telle que la diisopropyl éthylamine, pour accélérer la réaction.

On peut séparer le dérivé de formule (III) ainsi obtenu du milieu réactionnel par évaporation du solvant et par précipitation. On peut renouveler l'étape de précipitation et purifier ensuite le dérivé par chromatographie échangeuse d'ions afin d'éliminer la cyclodextrine naturelle n'ayant pas réagi. La précipitation peut être effectuée en ajoutant le mélange réactionnel concentré dans un solvant organique tel que l'acétone, et en séparant ensuite le précipité formé par filtration ou centrifugation. On parvient ainsi à éliminer totalement les dérivés de l'acide sulfonique ainsi que la diamine présente en excès et la diisopropyléthylamine qui sont tous solubles dans un solvant organique tel que l'acétone.

Dans l'étape b), on fait réagir le dérivé de formule (III) avec le réactif voulu, par exemple un dicarbonate de ditert-butyle lorsque R¹ représente le groupe tert-butyloxycarbonyle, dans un solvant organique tel que le DMF.

Lorsque R¹ représente le groupe nitrophényle sulfényle, le réactif utilisé peut être le chlorure de nitrophényl sulfényle.

Lorsque R¹ représente le groupe fluorène-9-yl-méthoxy carbonyle, le réactif utilisé peut être le fluorène-9-yl-méthoxy succinimidyl carbonate.

Lorsque R¹ est le groupe benzyloxycarbonyle ou un groupe de formule -COOR³, on peut utiliser pour cette réaction le chlorure ou l'azide de benzyloxycarbonyle.

Le dérivé de cyclodextrine de formule (I) ainsi obtenu peut être purifié par précipitations successives dans un solvant organique tel que l'acétone.

Le dérivé tosylé ou naphtosulfonylé de formule (II) utilisé comme produit de départ dans le procédé de l'invention peut être préparé par réaction de chlorure de p-toluène sulfonyle ou de chlorure de naphtosulfonyle sur la cyclodextrine correspondante en milieu aqueux.

Lorsque l'on veut préparer un dérivé de cyclodextrine répondant à la formule (I) donnée ci-dessus, dans laquelle un ou plusieurs R² représentent NH-(CH₂)ₘ-NHR¹, on peut partir des dérivés halogénés, bromés ou iodés, correspondants que l'on fait réagir avec le diaminoalcane de formule NH₂-(CH₂)ₘ-NH₂, puis effectuer comme précédemment une réaction en vue de protéger les groupes amines du dérivé de cyclodextrine par les groupes protecteurs voulus.

L'invention a encore pour objet les complexes d'inclusion du dérivé de cyclodextrine de formule (I) avec un composé chimique hydrophobe telle qu'une molécule pharmaceutiquement active.

Les composés chimiques hydrophobes susceptibles d'être inclus dans le dérivé de cyclodextrine de l'invention peuvent être de différents types. A titre d'exemple de tels composés on peut citer des produits cosmétiques, des vitamines, des molécules pharmaceutiquement actives telles que celles décrites par D. Duchêne dans l'ouvrage intitulé "Cyclodextrins and their industrial uses" précédemment mentionné.

De préférence dans l'invention, le composé chimique hydrophobe est une molécule pharmaceutiquement active.

A titre d'exemple de telles molécules, on peut citer les composés aromatiques de la famille des anthracyclines.

Comme on l'a indiqué plus haut, les dérivés de cyclodextrines de l'invention sont particulièrement intéressants en vue de leur incorporation, notamment sous la forme de complexes d'inclusion contenant des molécules hydrophobes, dans des systèmes organisés de tensioactifs.

Aussi, l'invention a aussi pour objet un système de tensioactif organisé comprenant un dérivé de cyclodextrine ou un complexe d'inclusion de cé dérivé conformes à l'invention.

Les tensioactifs susceptibles de former de tels systèmes organisés peuvent être de différents types. A titre d'exemple, on peut citer les phospholipides répondant à la formule générale suivante :

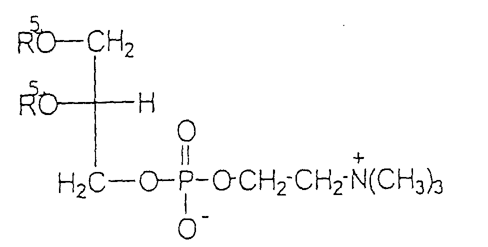

R⁵ = CH₃(CH₂)ₚCO-

dans laquelle R⁵ représente CH₃(CH₂)ₚ C0- avec p étant un nombre entier allant de 6 à 18.

Ces phospholipides sont capables de former de petites vésicules unilamellaires. C'est le cas en particulier du dimyristoylphosphatidylcholine (DMPC) qui répond à la formule ci-dessus avec p = 12.

Pour incorporer le complexe d'inclusion dans le système organisé de tensioactif, on peut dissoudre le complexe dans l'eau, puis ajouter le tensioactif et soumettre l'ensemble à une sonication.

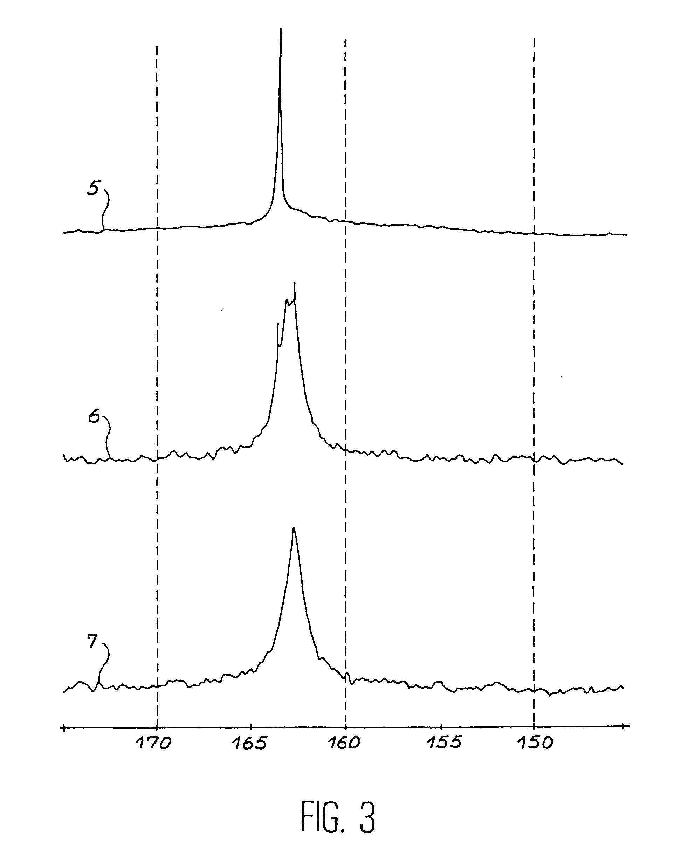

D'autres caractéristiques et avantage de l'invention apparaîtront mieux à la lecture des exemples suivants donnés bien-entendu à titre illustratif et non limitatif, en référence aux figures 1 à 3 annexées.

Les figures 1 et 2 illustrent les spectres de résonance magnétique nucléaire du proton du dérivé de cyclodextrine obtenu dans l'exemple 1 (spectre 1), du complexe d'inclusion obtenu dans l'exemple 2 (spectres 2 et 4), et de l'ASANA seul (spectre 3).

La figure 3 illustre les spectres de résonance magnétique nucléaire du ³¹P du complexe d'inclusion de l'invention dans le DMPC (spectre 5), du DMPC avec le dérivé de cyclodextrine de l'exemple 1 (spectre 6) et du DMPC seul (spectre 7).

### EXEMPLE 1 : Préparation du mono-6-désoxy-6-N-Boc-diamino-hexane-cyclomaltoheptaose.

### a) Préparation du mono-6-dèsoxy-6-p-toluène sulfonyl-cyclomaltoheptaose.

60 g de cyclomaltoheptaose (52,8 mmol) sont mis en suspension dans 500 ml d'eau distillée. On ajoute goutte à goutte 6,57 g (164 mmol) de soude caustique dissoute dans 20 ml d'eau sur 5 minutes avec forte agitation magnétique. A la solution limpide obtenue sont ajoutés 10,08 g (52,9 mmol) de chlorure de p-toluène sulfonyle dans 30 ml d'acétonitrile goutte à goutte sur 10 minutes. Après 2 h d'agitation à température ambiante, le précipité formé est éliminé par filtration et le filtrat est conservé 48 h à 4°C. Le précipité est isolé par filtration sous vide, lavé par 50 ml d'eau glacée et recristallisé immédiatement dans l'eau bouillante. Après une nuit à 4°C, le précipité est filtré et séché sous vide à 30°C. On obtient ainsi 7,5 g (rendement = 12 %) d'un composé pur conforme aux spécifications.

### b) Synthèse du mono-6-désoxy-6-diamino-hexanecyclomaltoheptaose.

Dans un ballon de 250 ml, surmonté d'un réfrigérant, on introduit 450 mg (3,87 mmol) de diaminohexane en solution dans 15 ml de DMF. On ajoute goutte à goutte 400 µl (2,33 mmol) de diisopropyléthylamine et on laisse sous agitation pendant 10 min. 1 g (0,776 mmol) du mono-6-désoxy-6-p-toluène sulfonyl-cyclomaltoheptaose obtenu précédemment en solution dans 10 ml de DMF est ajouté goutte à goutte à la solution précédente. Le mélange réactionnel est maintenu 3 jours sous agitation et à 75°C. Le solvant est éliminé sous pression réduite à 40°C. L'huile résiduelle est reprise par 5 ml d'eau, puis traitée par 150 ml d'acétone. Après une nuit à 4°C, le précipité est isolé, lavé à l'acétone et séché sous vide à 30°C. On obtient 700 mg (rendement de 73 %) de mono-6-désoxy-6-diamino-hexane-cyclomaltoheptaose. L'amino-cyclodextrine obtenue contient toujours une faible proportion de cyclodextrine non modifiée provenant de l'étape de tosylation. Cette dernière est éliminée facilement par une chromatographie sur résine échangeuse d'ion comme suit. Une solution de mono-6-désoxy-6-diamino-hexane-cyclomaltoheptaose (700 mg) dans 10 ml d'eau est appliquée à une colonne contenant 70 ml de résine Lewatit® SP 1080 sous forme H⁺ (Merck) en suspension dans l'eau. Après lavage par 200 ml d'eau, la mono-6-désoxy-6-diamino-hexane-cyclomatoheptaose est éluée par une solution aqueuse d'ammoniaque à 6 %. 200 ml de l'éluat basique sont collectés et concentrés sous vide à 30°C. Les dernières traces d'ammoniaque sont éliminées par évaporation sous vide en présence de 20 ml d'eau. Le résidu est repris dans un minimun d'eau et est précipité par 80 ml d'acétone, maintenu à 4°C pendant une nuit, isolé par filtration, lavé à l'acétone et séché sous vide à 30°C. On obtient ainsi 580 mg de composé final totalement débarassé de la cyclodextrine d'origine.
MS : m/z = 1233 [M+H]⁺ (M = 1232 calc pour C₄₈N₂O₃₄H₈₄) ;
RMN(¹³C, 125 MHz, d6-DMSO) : 106 ppm (C-1), 88 et 85,5 ppm (C-4) 77-74,5 ppm (C-2, C-3 et C-5), 63,8 ppm (C-6), 54 ppm (-NH-CH₂ et CH₂-NH₂), 33,6-30,5 ppm (4 CH₂).

### c) Synthèse du mono-6-désoxy-6-N-Boc-diamino-hexane-cyclomaltoheptaose.

A 50 mg (0,041 mmol) de mono-6-désoxy-6-diamino-hexane-cyclomaltoheptaose dans 5 ml de DMF, on ajoute 12 µl (0,049 mmol) de ditertiobutylbicarbonate à 0°C et sous agitation. On laisse revenir à température ambiante et sous agitation pendant 2 jours et on élimine le solvant sous pression réduite à 30°C jusqu'à l'obtention d'une huile. Celle-ci est traitée par 75 ml d'acétone. Après une nuit à 4°C, le précipité est isolé, lavé à l'acétone et séché sous vide à 30°C. On obtient 46 mg (rendement de 86%) de mono-6-désoxy-6-N-Boc-diamino-hexane-cyclomaltoheptaose.
MS : m/z = 1333 [M+M]⁺ (M = 1332 calc pour C₅₃H₉₂O₃₆N₂) ;
IR : ν = 1665 cm⁻¹ (NHCO);
RMN (¹H, 500 MHz, D20), 5,05-5,20 ppm (H-1), 4,13 ppm (H-6A), 3,85-3,95 ppm (H-3), 3,65-3,74 ppm (H-2) 3,60-3,78 ppm (H-4), 3,41 ppm (H-4A) 4,29, 4,05, 3,95-3,75 et 3,63 ppm (H-5), 3,85 et et 3,72 ppm (CH-a' et CH-b'), 3,24 ppm (CH₂-C), 2,97 et 2,91 ppm (CH-a" et CH-b"), 1,60 ppm (CH₂-d), 1,40 ppm (CH₂-e), 1,30 ppm (CH₂-f), 1,52 ppm ((CH₃)₃).

### EXEMPLE 2 : Préparation d'un complexe d'inclusion du mono-6-désoxy-6-N-Boc-diamino-hexane-cyclomaltoheptaose et du sel de sodium de l'acide anthraquinonesulfonique(ASANA).

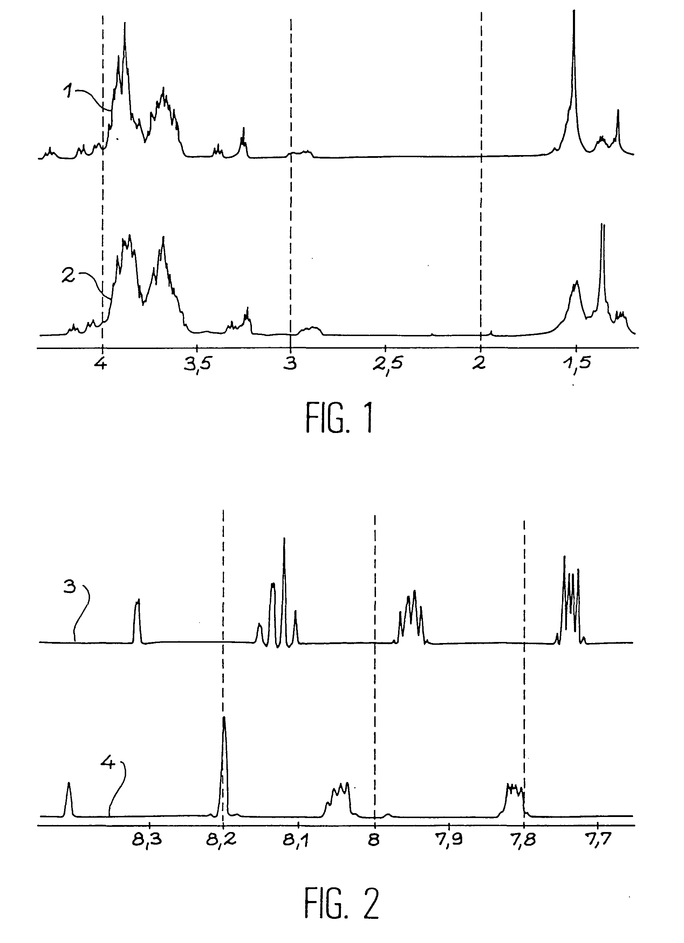

On prépare le complexe d'inclusion de l'ASANA en formant une solution aqueuse comprenant 6 mol/l d'ASANA et 6 mol/l de mono-6-désoxy-6N-Boc-diamino-hexane-cyclomaltogheptaose. On examine ensuite la solution par spectrométrie par RMN du proton à 500 MHz et à 298 K dans D₂O. Le spectre obtenu dans ces conditions est représenté sur les figures 1 et 2 (spectres 2 et 4). Sur ces figures, on a également représenté le spectre de la cyclodextrine de l'exemple 1 seule (spectre 1) et de l'ASANA seule (spectre 3).

Sur la figure 1, le spectre 1 est le spectre obtenu avec une solution aqueuse à 6 mol/l du dérivé de cyclodextrine seule.

Si l'on compare les spectres 1 et 2 de la figure 1, on remarque que l'analyse des déplacements chimiques des différents protons montre une variation des déplacements chimiques des protons H-3 et H-5 de la β-cyclodextrine modifiée par inclusion.

Sur la figure 2 (le spectre 3) est le spectre obtenu avec une solution aqueuse contenant uniquement 6 mol/l d' ASANA.

Si l'on compare les deux spectres 3 et 4, le déplacement des protons aromatiques de l'ASANA met en évidence l'inclusion de celui-ci dans la cavité du dérivé de cyclodextrine.

### EXEMPLE 3 : Incorporation du complexe d'inclusion de l'exemple 2 à de petites vésicules unilamellaires de dirnyristoylphosphatidylcholine (DPMC).

On prépare une solution (CHCl₃/MeOH 85 : 15) 15 mM de DMPC. On évapore le solvant sous pression réduite à 25°C et on ajoute ensuite 500 µl d'eau. On

## Revendications

1. Dérivé de cyclodextrine répondant à la formule : 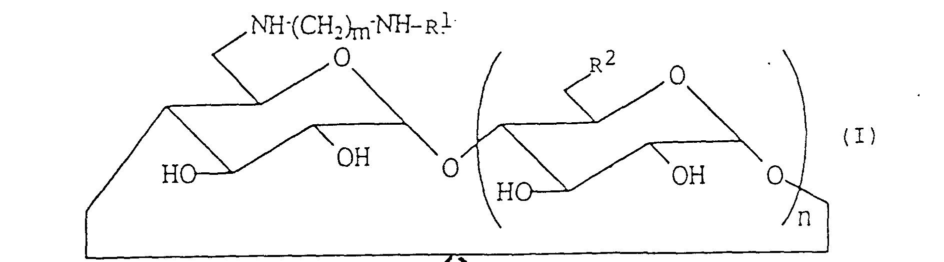 dans laquelle R¹ représente le groupe nitrophénylsulfényle, le groupe fluorène-9-yl-méthoxy carbonyle, ou un groupe de formule -COOR³ dans laquelle R³ est un groupe alkyle ou aryle éventuellement substitué, les R² qui peuvent être identiques ou différents, représentent OH ou NH-(CH₂)ₘ-NHR¹, m est un nombre entier allant de 2 à 18, et n est égal à 5, 6 ou 7.

2. Dérivé selon la revendiation 1, **caractérisé en ce que** R¹ est le groupte t-butyl oxycarbonyle.

3. Dérivé de cyclodextrine selon l'une quelconque des revendications 1 à 2, **caractérisé en ce que** tous les R² représentent OH.

4. Dérivé de cyclodextrine selon l'une quelconque des revendications 1 à 3, **caractérisé en ce que** n est égal à 6.

5. Dérivé de cyclodextrine selon l'une quelconque des revendications 1 à 4, **caractérisé en ce que** m est égal à 6.

6. Procédé de préparation d'un dérivé de cyclodextrine répondant à la formule : agite le tout au moyen d'un vortex afin d'obtenir une suspension homogène et on lyophilise. A la solution de complexe décrite dans l'exemple 2 ou à une solution à 6 mmol/l du dérivé de cyclodextrine seule de l'exemple 1, on ajoute la DMPC lyophilisée. L'ensemble est d'abord agité, puis soniqué pendant 20 min jusqu'à l'obtention d'une solution transparente.
On examine ensuite la solution par spectrométrie RMN du ³¹P à 81 MHz. Le spectre obtenu dans ces conditions est représenté sur la figure 3 (spectre 5).
Sur cette figure, le spectre 6 est celui obtenu lorsqu'on utilise une solution à 6 mmol/l du dérivé de cyclodextrine seul de l'exemple 1 au lieu de la solution du complexe d'inclusion de l'exemple 2, et le spectre 7 est celui de la solution de départ à 15 mmol/l de DMPC.
En comparant ces spectres, on peut voir que l'inclusion de l'ASANA dans la cavité du dérivé de cyclodextrine favorise l'incorporation de la chaîne hydrophobe dans les petites vésicules unilamellaires de DMPC.
Ces vésicules de DMPC sont des modèles de membranes biologiques. L'incorporation des chaînes aliphatiques du dérivé de cyclodextrine conduit à des structures de type vésiculaire tapissées de cavités de cyclodextrine restant à l'extérieur qui peuvent inclure des molécules exogènes hydrophobes de façon spécifique.
Ainsi, les dérivés de cyclodextrine de l'invention peuvent être incorporés à de petites vésicules unilamellaires de phospholipides tout en conservant leur capacité d'inclusion de molécules hydrophobes. 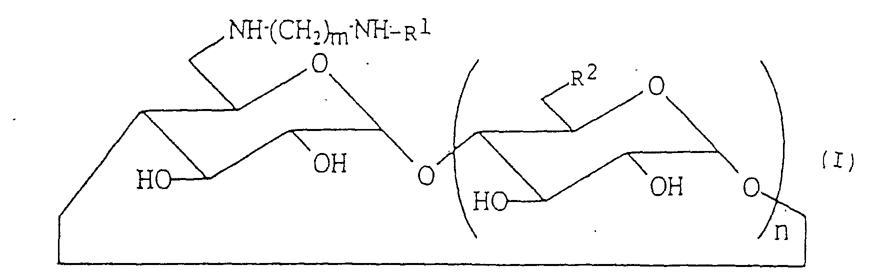 dans laquelle R¹ représente un groupe protecteur d'une fonction amine, les R² représentent OH, m est un nombre entier allant de 2 à 18, et n est égal à 5, 6 ou 7, **caractérisé en ce qu'**il comprend les étapes suivantes :
a) faire réagir un dérivé de cyclodextrine de formule : 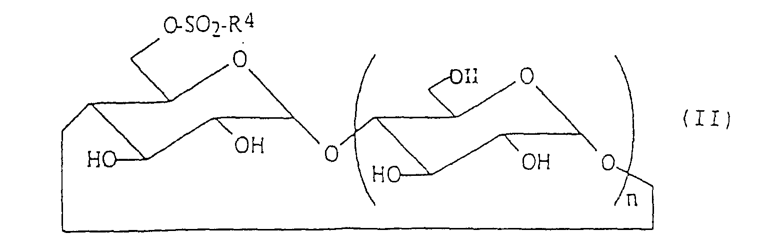 dans laquelle R⁴ est un groupe p-tolyle ou naphtyle, et n est égal à 5, 6 ou 7, avec un diamino alcane de formule NH₂-(CH₂)ₘ-NH₂ dans laquelle m a la signification donnée ci-dessus, pour obtenir un dérivé de cyclodextrine de formule : 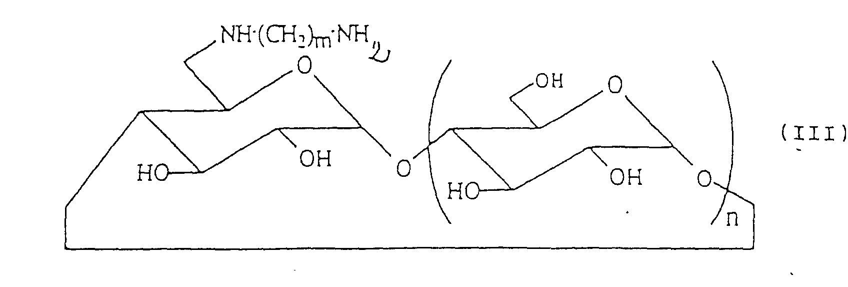 dans laquelle n et m sont tels que définis ci-dessus, et
b) faire réagir le dérivé de formule (III) avec un réactif comportant le groupe protecteur R¹ pour obtenir le dérivé de cyclodextrine de formule (I).

7. Complexe d'inclusion d'un dérivé de cyclodextrine selon l'une quelconque des revendications 1 à 5 avec un composé chimique hydrophobe.

8. Complexe selon la revendication 7, **caractérisé en ce que** le composé chimique hydrophobe est une molécule pharmaceutiquement active.

9. Complexe selon la revendication 8, **caractérisé en ce que** la molécule pharmaceutiquement active est une anthracycline.

10. Système Système de tensioactif organisé comprenant un dérivé de cyclodextrine selon l'une quelconque des revendications 1 à 5 ou un complexe d'inclusion selon l'une quelconque des revendications 7 à 9.

11. Système selon la revendication 9, **caractérisé en ce que** le tensioactif est un phospholipide.

## Patentansprüche

1. Cyclodextrin-Derivat entsprechend der Formel: 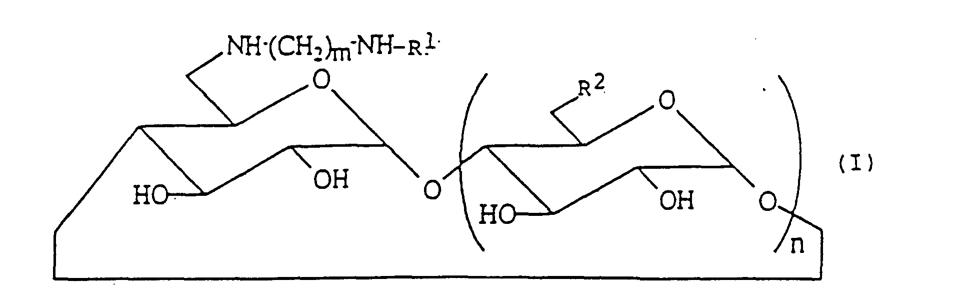 in welcher R¹ die Nitrophenylsulfenylgruppe, die Fluoren-9-yl-methoxycarbonylgruppe oder eine Gruppe der Formel -COOR³, worin R³ eine gegebenenfalls substituierte Alkyl- oder Arylgruppe ist, darstellt, die R², die identisch oder verschieden sein können, OH oder NH-(CH₂)ₘ-NHR¹ darstellen, m eine ganze Zahl ist, die von 2 bis 18 geht, und n gleich 5, 6 oder 7 ist.

2. Derivat nach Anspruch 1, **dadurch gekennzeichnet, dass** R¹ die tert-Butyloxycarbonylgruppe ist.

3. Cyclodextrin-Derivat nach einem der Ansprüche 1 bis 2, **dadurch gekennzeichnet, dass** alle R² OH darstellen.

4. Cyclodextrin-Derivat nach einem der Ansprüche 1 bis 3, **dadurch gekennzeichnet, dass** n gleich 6 ist.

5. Cyclodextrin-Derivat nach einem der Ansprüche 1 bis 4, **dadurch gekennzeichnet, dass** m gleich 6 ist.

6. Verfahren zur Herstellung eines Cyclodextrin-Derivats entsprechend der Formel: 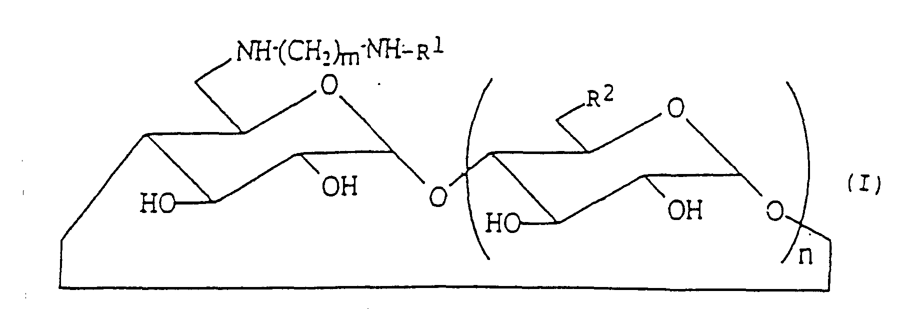 in welcher R¹ eine Schutzgruppe für eine Aminofunktion darstellt, die R² OH darstellen, m eine von 2 bis 18 gehende ganze Zahl ist und n gleich 5, 6 oder 7 ist,
**dadurch gekennzeichnet, dass** es die folgenden Schritte umfasst:
a) ein Cyclodextrin-Derivat der Formel: 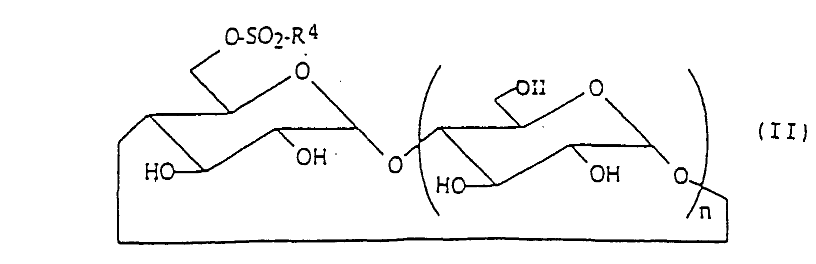 in welcher R⁴ eine p-Tolyl- oder Naphthylgruppe ist und n gleich 5, 6 oder 7 ist, mit einem Diamino-alkan der Formel NH₂-(CH₂)ₘ-NH₂, in welcher m die oben gegebene Bedeutung hat, reagieren zu lassen, um ein Cyclodextrin-Derivat der Formel: 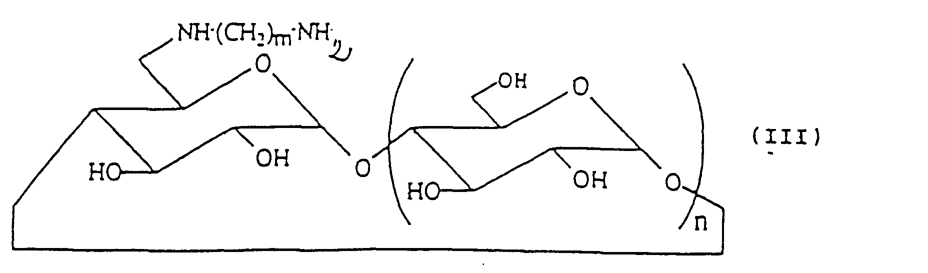 zu erhalten, in welcher n und m wie oben definiert sind, und
b) das Derivat der Formel (III) mit einem Reagenz reagieren zu lassen, das die Schutzgruppe R¹ enthält, um das Cyclodextrin-Derivat der Formel (I) zu erhalten.

7. Einschlusskomplex eines Cyclodextrin-Derivats nach einem der Ansprüche 1 bis 5 mit einer hydrophoben chemischen Verbindung.

8. Komplex nach Anspruch 7, **dadurch gekennzeichnet, dass** die hydrophobe chemische Verbindung ein pharmazeutisch aktives Molekül ist.

9. Komplex nach Anspruch 8, **dadurch gekennzeichnet, dass** das pharmazeutisch aktive Molekül ein Anthracyclin ist.

10. Organisiertes Tensidsystem, umfassend ein Cyclodextrin-Derivat nach einem der Ansprüche 1 bis 5 oder einen Einschlusskomplex nach einem der Ansprüche 7 bis 9.

11. System nach Anspruch 9, **dadurch gekennzeichnet, dass** der grenzflächenaktive Stoff ein Phospholipid ist.

## Claims

1. Cyclodextrin derivative according to formula: 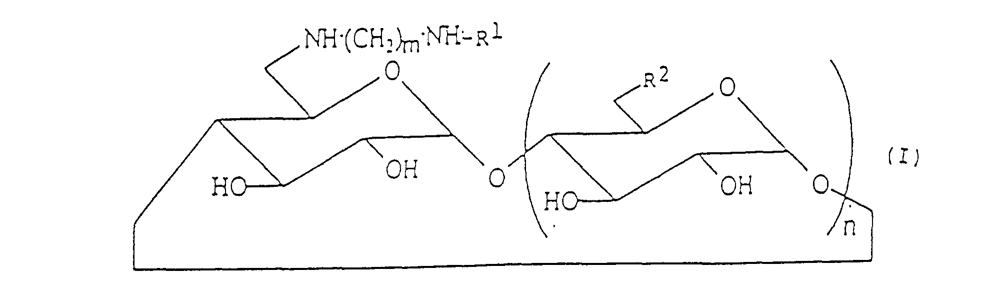 in which R¹ represents the nitrophenyl sulphenyl group, the fluoren-9-yl-methoxycarbonyl group, or a group of formula -COOR³ in which R³ is an alkyl group or an aryl group, optionally substituted, the R², which can be the same or different, represent OH or NH-(CH₂)ₘ-NHR¹, m is an integer from 2 to 18 and n is equal to 5, 6 or 7.

2. Derivative according to claim 1, **characterized in that** R¹ is the t-butyl oxycarbonyl group.

3. Cyclodextrin derivative according to either of the claims 1 and 2, **characterized in that** all the R² represent OH.

4. Cyclodextrin derivative according to any one of the claims 1 to 3, **characterized in that** n is equal to 6.

5. Cyclodextrin derivative according to any one of the claims 1 to 4, **characterized in that** m is equal to 6.

6. Process for the preparation of a cyclodextrin derivative according to the formula: 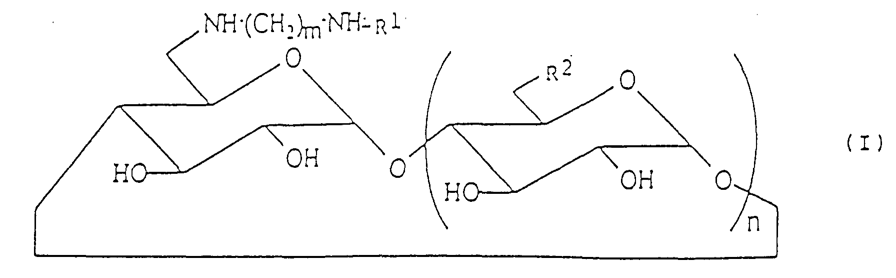 in which R¹ represents a group protecting an amine function, the R² represent OH, m is an integer from 2 to 18 and n is equal to 5, 6 or 7, **characterized in that** it comprises the following stages:
a) reacting a cyclodextrin derivative of formula: 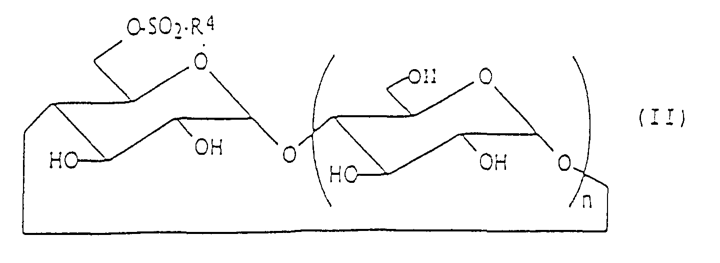 in which R⁴ is a p-tolyl or naphthyl group, and n is equal to 5, 6 or 7, with a diaminoalkane of formula NH₂-(CH₂)ₘ-NH₂, in which m has the meaning given hereinbefore, in order to obtain a cyclodextrin derivative of formula: 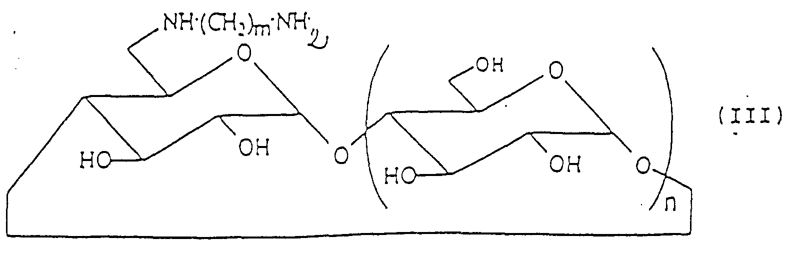 in which n and m are as defined hereinbefore and
b) reacting the derivative of formula (III) with a reagent incorporating the protecting group R¹ to obtain the cyclodextrin derivative of formula (I).

7. Inclusion complex of a cyclodextrin derivative according to any one of the claims 1 to 5 with a hydrophobic chemical compound.

8. Complex according to claim 7, **characterized in that** the hydrophobic chemical compound is a pharmaceutically active molecule.

9. Complex according to claim 8, **characterized in that** the pharmaceutically active molecule is anthracyclin.

10. Organized surfactant system incorporating a cyclodextrin derivative according to any one of the claims 1 to 5 or an inclusion complex according to any one of the claims 7 to 9.

11. System according to claim 9, **characterized in that** the surfactant is a phospholipid.
